# EUROPEAN PATENT SPECIFICATION

(11) **EP 2 489 303 B1**
(45) Date of publication and mention of the grant of the patent: **28.03.2018**
(21) Application number: 10822990.7
(22) Date of filing: 15.10.2010
(51) Int. Cl.: A61B 5/06, G01R 33/02

(54) **POSITIONING SYSTEM AND METHOD FOR ESOPHAGEAL PH VALUE WIRELESS MONITORING**
POSITIONIERUNGSSYSTEM UND -VERFAHREN ZUR DRAHTLOSEN ÜBERWACHUNG DES SPEISERÖHREN-PH-WERTES
SYSTÈME DE POSITIONNEMENT ET PROCÉDÉ POUR LA SURVEILLANCE SANS FIL DE LA VALEUR DU PH OESOPHAGIEN

(30) Priority: 16.10.2009 CN 200910191195
(43) Date of publication of application: 22.08.2012
(73) Proprietor: Chongqing Jinshan Science & Technology (Group) Co., Ltd., Chongqing 401120 (CN)
(72) Inventor: LI, Xiangdong, Chongqing 401120 (CN); YUAN, Jian, Chongqing 401120 (CN); QIN, Lang, Chongqing 401120 (CN); TONG, Wanli, Chongqing 401120 (CN); GONG, Zhaotao, Chongqing 401120 (CN)
(74) Representative: Groth & Co. KB
(86) International application number: PCT/CN2010/001623
(87) International publication number: WO 2011/044758

(56) References cited:
- EP-A1- 1 676 522
- EP-A1- 1 875 852
- WO-A2-03/005877
- CN-A- 1 929 775
- CN-A- 101 108 122
- CN-A- 101 406 410
- CN-A- 101 711 673
- CN-U- 201 505 133
- CN-Y- 201 275 065
- US-A1- 2003 073 935
- US-A1- 2006 231 110
- US-A1- 2008 177 136
- US-A1- 2008 242 931

## Description

### Field of the Invention

The invention refers to a positioning system, apparatus, and method for wireless monitoring of esophageal pH value.

### Background of the Related Art

As the pace of life of people speeds up and diet composition of people changes, the incidence of gastrointestinal functional diseases increases day by day, wherein the common esophageal diseases can be reflux esophagitis, esophageal cancer, esophageal stenosis, esophageal varices, dyspepsia, functional dysphagia, and this has brought suffering to the people physically and mentally. In the course of medical diagnosis and treatment, a continuous monitoring or treatment of specific three-dimensional space of the esophagus is often required, for example, identification for reflux esophagitis to determine if there is acid reflux or alkaline reflux and also determine the degree of regurgitation; for the esophageal disease, the treatments such as continuous electrical stimulation and drug release are applied; especially there is a need for understanding the effect of surgery after the esophageal surgery, so as to make sure if there is a rejection and if the organ function recovers, and there is a further need for long-term monitoring.

There are already many medical equipments for diagnosis and treatment for esophageal, for example, the push-type upper digestive tract endoscopies, such as gastroscopy, electronic gastroscope, endoscopic ultrasonography, which may enter the esophagus, observe the region of lesion, acquire the image, and conduct resection. However, it is not suitable for a long-period operation, as the tolerance of patient to the above-mentioned endoscope is poor. The existing technologies include catheter-type physiological parameter monitor, such as catheter-type pH meter, catheter-type manometer, catheter-type bilirubin meter; catheter indwelling is required for these technologies, which brings pain to people, makes people embarrassment, and unable to eat, and it is also difficult for long time use.

Recently, a variety of radio telemetries have been invented and applied. An example is described in the patent application US 2008/0177136 A1, which relates to a method of detecting a swallowable capsule camera when entering into or exiting the GI tract. Generally, the existing capsule-shaped internal miniature device moves with the digestive peristalsis after being swallowed, and may acquire the image, and detect the parameters of the digestive tract such as pH value, and pressure; data are transmitted to the external miniature receiver via radio frequency, but it is still unable to realize the long term monitoring of specific three-dimensional space, as it is unable to fix the capsule-shaped internal miniature device.

Currently, in the market, there is a system for wireless monitoring of esophageal pH value, comprising a pH capsule, a data recorder, an analysis software and a fixture, to a certain extent, which to some extent overcomes the above mentioned shortcomings; for the system, a pin is used to fix the pH capsule on the esophageal wall, and the recorded pH data are wirelessly transmitted to the receiver at the waist of patient, so that there is no electrode lien of catheter.

Another example is disclosed in the patent application EP 1875852 A1 which relates to an in-vivo information acquiring apparatus capable of recording information inside a subject by using a portable recording medium. Other types of in-vivo examining devices can be found in WO 03/005877 A2.

However, the system also has the following shortcomings:
firstly, it is unable to understand the fixing condition of pH capsule in real time, so as not to be able to avoid the invalid detection due to the unexpected drop of the capsule, thereby increasing the cost of monitoring, meanwhile, it is also unable to ensure the testing accuracy of the pH capsule, due to the unexpected drop of the capsule;
secondly, as the testing of the system usually lasts for 24-48 hours, it is inevitable for the patients to change the position of the data recorder when they are working or sleeping, which may cause interruption of the signal so as to affect the integrity of testing.

Furthermore, as the storage time and environmental temperature vary, the testing accuracy of the pH sensor may vary, which affects the testing accuracy.

Similar systems are known such as the document US 2008/242931A1 which provides capsulated medical equipment for detecting whether a capsulated body is clogged at a sensed region in a body cavity or the document US 2003/073935 disclosing a capsule endoscopic system capable of reading an image of a target region in detail by adjusting the frame rate.

### Summary of the Invention

In view of the above-described problem, it is one object of the invention to provide a positioning system for wireless monitoring of esophageal pH value, as defined in independent claim 1, and it is a further object of the invention to provide a method for wireless monitoring of esophageal pH value, as defined in independent claim 4. The positioning system for wireless monitoring of esophageal pH value comprises an internal transmitting apparatus, and an external recording apparatus; the internal transmitting apparatus comprises a pH sensor, a sample circuit, a first micro-processor, a power management unit, a first wireless transceiver module, and a first means; the external recording apparatus comprises a second micro-processor, a power management unit, a buzzer, a storage, a data interface, a key, a second wireless transceiver module, a status light, a housing, and a second means; the second means of the external recording apparatus cooperates with the first means of the internal transmitting apparatus; if the external recording apparatus detects that the internal transmitting apparatus is not in the preset region, the micro-processor controls the buzzer and/or status light to alert; the external recording apparatus periodically detects the intensity of the signal received by second wireless transceiver module, under the control of the micro-processor, and if it is detected that the signal intensity is not in the preset range of signal intensity, the micro-processor controls the buzzer and/or the status light 208 to alert.

In a class of this embodiment, the first means of the internal transmitting apparatus may be a permanent magnet; the second means of the external recording apparatus may be a magnetic sensor; the cooperation between the second means of the external recording apparatus and the first means of the internal transmitting apparatus comprises the fact that the intensity of magnetic field generated by the permanent magnet of the internal transmitting apparatus is detected through the magnetic sensor, and if the intensity of magnetic field is not in the preset range of magnetic intensity, the external recording apparatus detects that the internal transmitting apparatus is not in the preset region.

In a class of this embodiment, the first means of the internal transmitting apparatus may be a reed switch in series with the pH sensor the sample circuit and arranged therebetween; the second means of the external recording apparatus may be a magnet, which actuates the reed switch through magnetic induction; and the cooperation between the second means of the external recording apparatus and the first means of the internal transmitting apparatus comprises the fact that if the actual distance between the magnet and the reed switch is larger than preset distance, the reed switch is actuated to open the circuit between the pH sensor and the sample circuit of the internal transmitting apparatus, and the external recording apparatus detects that the internal transmitting apparatus is not in the preset region. According to the invention, the external recording apparatus further comprises temperature sensor; and the storage pre-stores the first pH calibration data of the internal transmitting apparatus; and the temperature sensor detects the current room temperature, and sends the temperature data to the second micro-processor; and the second wireless transceiver module receives the initialized data from the internal transmitting apparatus and sends the data to the second micro-processor; and the second micro-processor calibrates the initialized data, and conducts temperature compensation in the course of calibration, in order to obtain the current calibration data; after that the second micro-processor compares the current calibration data with the first pH calibration data pre-stored in the storage; if the current calibration data differ from the first pH calibration data, a calibration alerting signal is sent to the internal transmitting apparatus through second wireless transceiver module; and the internal transmitting apparatus further comprises a work light; and the first wireless transceiver module of the internal transmitting apparatus receives the calibration alerting signal and send the signal to the first micro-processor; and the first micro-processor controls the work light to alert. The present disclosure describes furthermore an unclaimed internal transmission apparatus, comprising a pH sensor, a sample circuit, a first micro-processor, a power management unit, and a first wireless transceiver module; wherein, the pH sensor, the sample circuit, the first micro-processor, the first wireless transceiver module are successively connected together; the power management unit is connected respectively with the pH sensor, the sample circuit, the first micro-processor, and the first wireless transceiver module; the sample circuit, the first micro-processor, power management unit, and the first transceiver module are enclosed in a capsule housing; and the sensing portion of the pH sensor exposes outside the capsule housing, and may contact the body fluid in the esophagus; wherein, the internal transmitting apparatus further comprises a first means, disposed inside the capsule housing.

In a class of this unclaimed apparatus, the first means may be a permanent magnet, or a reed switch that is in series with the pH sensor and the sample circuit and arranged therebetween.

In a class of this unclaimed apparatus, further comprises a work light, which is connected with a first micro-processor 303 and receives the control signal from the first micro-processor to alert. The present disclosure describes furthermore an unclaimed external recording apparatus, wherein, comprising a second micro-processor, a power management unit, a buzzer, a storage, a data interface, a key, a second wireless transceiver module, a status light, a housing, and a second means; the second means cooperates with the first means of the internal transmitting apparatus; if it is detected that the internal transmitting apparatus is not in the preset region, the second micro-processor controls the buzzer and/or the status light to alert; the external recording apparatus periodically detects the intensity of the signal received by the second transceiver module under the control of the second micro-processor, and if it is detected that the signal intensity is not in the preset range of intensity, the second micro-processor controls the buzzer and/or the status light to alert.

In a class of this unclaimed apparatus, the external recording apparatus further comprises a temperature sensor, wherein, the storage pre-stores first pH calibration data of the internal transmitting apparatus; the temperature sensor detects the current room temperature, and sends the temperature data to the second micro-processor; the second wireless transceiver module receives the initialized data from the internal transmitting apparatus and sends the data to the second micro-processor; the second micro-processor calibrates the initialized data, and conducts temperature compensation in the course of calibration, in order to obtain the current calibration data, after that the second micro-processor compares the current calibration data with the first pH calibration data pre-stored in the storage; if the current calibration data differ from the first pH calibration data, a calibration alerting signal is sent to the internal transmitting apparatus through the second wireless transceiver module.

In accordance with a further embodiment of the invention provided is a positioning method for wireless monitoring of esophageal pH value, by means of an external recording apparatus and an internal transmitting apparatus, comprising determining if the internal transmitting apparatus locates in the preset region, through the cooperation between the recording apparatus and the internal transmitting apparatus, and alerting if the internal transmitting apparatus is not in the preset region; and determining if the signal intensity is in the preset range of intensity, based on the intensity of the received signal, which is periodically detected by the external recording apparatus, and alerting if the signal intensity is not in the preset range of signal intensity. According to the invention, the method, before the system is put in use, further comprises pre-storing the first pH calibration data of the internal transmitting apparatus in the external recording apparatus; and calibrating the initialized data received by the external recording apparatus from the internal transmitting apparatus, and conducting room temperature compensation in the course of calibration receives the initialized data, in order to obtain the current calibrated data; comparing the current calibrated data with the first pH calibration data pre-stored, and sending a calibration alerting signal to the internal transmitting apparatus if the current calibrated data differ from the first pH calibration data; and controlling the internal transmitting apparatus to alert after the internal transmitting apparatus receives the calibration alerting signal.

In the positioning system and method for wireless monitoring of esophageal pH value of this invention, the cooperation between the first means of the internal transmitting apparatus and the second means of the external recording apparatus enables the real time monitoring of the position of the internal transmitting apparatus in the esophagus, which not only avoids the invalid detection due to unexpected drop of the capsule, but also reduces the cost of detection for patients; in addition, the fact that the internal transmitting apparatus is applied for detecting the intensity of the signal received may help to avoid signal interrupt problem caused when the patients are working or sleeping, which ensures the integrality of detection data; furthermore, the calibration of the system before being put in use and the temperature compensation in the course of calibration improve the testing accuracy. Compared with the prior art, this invention has the advantages such as quicker acquisition of the positioning condition, more stability of signal, and more accuracy of detection, as well as ease of implementation, which is more acceptable to the doctors and the patients.

### Brief Description of the Drawings

Benefits and advantages of the present invention will become apparent to those skilled in the art to which this invention relates from the subsequent description of exemplary embodiments and the appended claims, taken in conjunction with the accompanying drawings, in which:
Fig.1 is a schematic diagram of the state of use of one embodiment of this invention;
Fig.2 is a circuit block diagram of the internal transmitting apparatus of one embodiment of this invention;
Fig.3 is a circuit block diagram of the external recording apparatus of one embodiment of this invention;
Fig.4 is a circuit block diagram of the second wireless transceiver module of the embodiment as illustrated in Fig.3;
Fig.5 is a flow diagram of positioning the internal transmitting apparatus of one embodiment of this invention;
Fig.6 is a flow diagram of implementation of alerting for communication failure of one embodiment of this invention;
Fig.7 is a flow diagram of calibration of the system of one embodiment of this invention before being put in use.

### Detailed Description of Embodiments

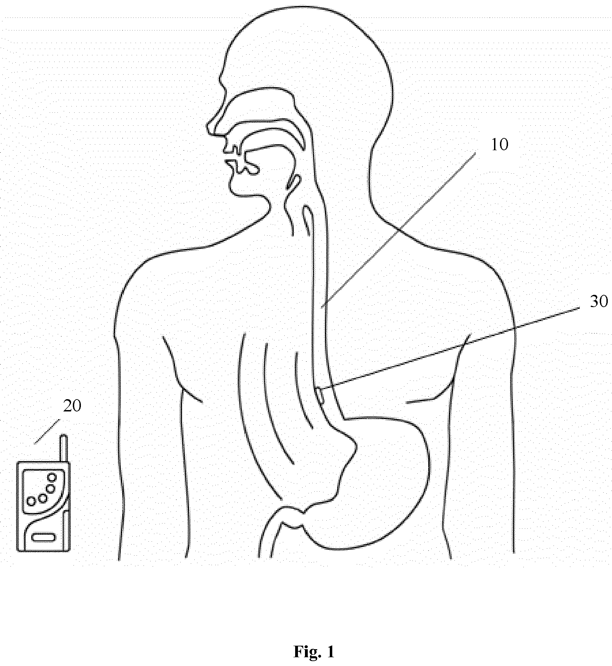

Preferred embodiments of the invention will now be described, by way of example only, with reference to the accompanying drawings. Referring to the embodiment shown on Fig.1, the internal transmitting apparatus 30 is fixed on the esophagus 10, and data are transmitted between the internal transmitting apparatus 30 and the external recording apparatus 20 through radio frequency technology. Wherein, the internal transmitting apparatus 30 may be a pH capsule, and the pH capsule is streamlined and with flat capsule shaped structure; the external recording apparatus 20 may be a data recorder.

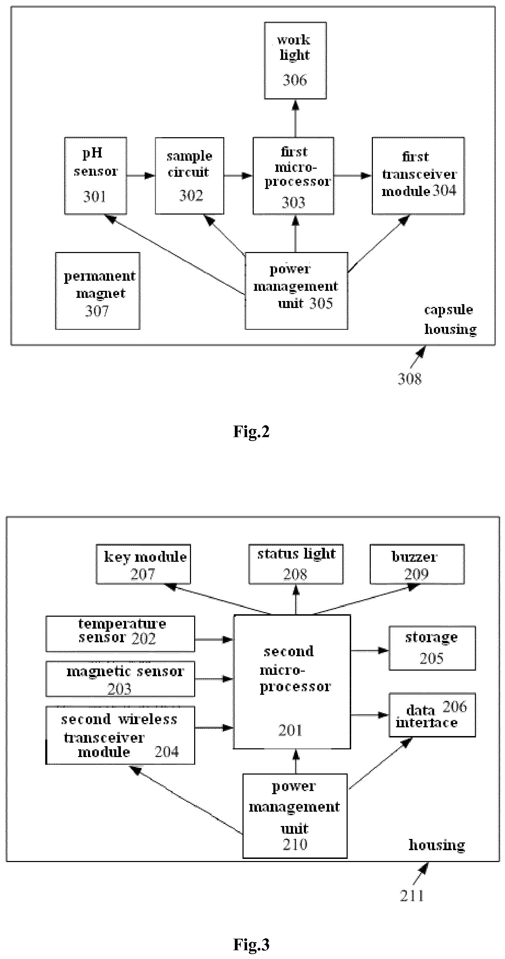

In Fig.2, the internal transmitting apparatus 30 of this embodiment is in the form of a pH capsule. The pH capsule comprises a pH sensor 301, a sample circuit 302, a first micro-processor 303, a power management unit 305, a first wireless transceiver module 304, and wherein, the pH sensor 301, the sample circuit 302, the first micro-processor 303, the first wireless transceiver module 304 are successively connected together; and the power management unit 305 is connected respectively with the pH sensor 301, the sample circuit 302, the first micro-processor 303, and the first wireless transceiver module 304; and the sample circuit 302, the first micro-processor 303, power management unit 305, and the first transceiver module 304 are enclosed in a capsule housing 308; and the sensing portion of the pH sensor 301 exposes outside the capsule housing 308, and may contact the body fluid in the esophagus; wherein, the pH capsule further comprises a first means 307, disposed inside the capsule housing 308.

In the pH capsule illustrated in Fig.2, under the control of the first micro-processor 303, the pH sensor 301 detects the pH value of the body fluid in the esophagus periodically, and the pH value is converted into digital data through the sample circuit 302 and stored temporarily in the first micro-processor 303 of the capsule, and after a certain period of time, the data packages are transmitted to the external data recorder through the first wireless transceiver module 304, and specifically, the external data recorder is the external recording apparatus 20. Wherein, the power management unit 305 may be the 3V silver oxide button cell; the first micro-processor 303 may be a chip with A/D unit and RAM built in; the pH sensor may consist of a medical antimony measuring electrode and Ag/AgCl reference electrode; the sample circuit 302 conducts impedance matching, signal amplification and signal filtering, after that the built-in A/D unit of the first micro-processor 303 acquires the data, and then the data are transmitted to the external data recorder through a first wireless transceiver module 304 using FSK/ASK communication technology and 433 MHz ISM European band. The first wireless transceiver module 304 comprises a power amplifier (PA).

Specifically, in the embodiment as illustrated in Fig.2, the first means 307 is in the form of a permanent magnet, which does not contact any component in the capsule housing 308. The material of the permanent magnet can be NdFeB, AlNiCo or other high magnetism materials; the permanent magnet is with schistose texture, and the direction of magnetization of the permanent magnet is in the width direction.

In addition, in the embodiment as illustrated in Fig.2, the system further comprises a work light 306 connected with the first micro-processor 303, which alerts after receiving the control signal from the first micro-processor 303.

Furthermore, in other embodiments, the first means 307 may be a reed switch that is in series with the pH sensor 301 and the sample circuit 302 and arranged therebetween.

In the embodiment as illustrated in Fig.3, the external recording apparatus may be in the form of data recorder. The data recorder comprises a second micro-processor 201, a power management unit 210, a buzzer 209, a storage 205, a data interface 206, a key 207, a second wireless transceiver module 204, a status light 208, a data recorder housing 211, and a second means 203; the above mentioned components are installed inside the data recorder housing 211 except the key 207. Wherein, the second wireless transceiver module 204 receives pH data from the pH capsule 30, and the pH data are temporarily stored in the storage 205, or exported through the data interface 206, under the control of the second micro-processor 201. The computer controls the calibration of time and pH value of the data recorder through the data interface 206. The housing 22 of the data recorder is made of the materials that do no harm to people's health; the patient may use the key 207 to record the event such as eating, sleeping, lying, and cardialgia, in the course of operating the system; the storage 205 of the data recorder is for storing the pH data, and the pH data can be transmitted to the data processor such as the computer via the data interface 16. The power management unit 210 can be three 7# Alkali dry batteries. The storage 205 may be nonvolatile storage such as Flash, Fram, and EEPROM. The status light 208 may be in the form of red, green, yellow LED or other displaying components.

The second means 203 in Fig.3 cooperates with the first means 307 of the internal transmitting apparatus 30; if it is detected that the internal transmitting apparatus 30 locates in the preset region, the data are sent by the internal transmitting apparatus 30 through the first wireless transceiver module 304; if it is detected that the internal transmitting apparatus 30 is not in the preset region, the second micro-processor 201 controls the buzzer 209 and/or the status light 208 to alert.

More specifically, the first means 307 of the internal transmitting apparatus 30 may be in the form of a permanent magnet, and the second means 203 of the external recording apparatus 20 may be in the form of a magnetic sensor for the detection of the magnetic field of the permanent magnet; and the cooperation between the second means 203 and the first means 307 of the internal transmitting apparatus 30 refers to the fact that if the intensity of the magnetic field is in the preset range of magnetic intensity, the external recording apparatus 20 detects that the internal transmitting apparatus 30 locates in the preset region; and if the intensity of the magnetic field is not in the preset range of magnetic intensity, the external recording apparatus 20 detects that the internal transmitting apparatus 30 is not in the preset region.

Alternatively, the first means 307 of the internal transmitting apparatus 30 may be in the form of a reed switch, and the second means 203 of the external recording apparatus 20 may be in the form of a magnet; the reed switch is actuated through magnetic induction; the cooperation between the second means 203 of the external recording apparatus and the first means 30 of the internal transmitting apparatus 30 refers to the fact that if the actual distance between the magnet and the reed switch is not larger than the preset distance, the reed switch is actuated to close the circuit between the pH sensor 301 and the sample circuit 302 of the internal transmitting apparatus 30, and the external recording apparatus 20 detects that the internal transmitting apparatus 30 locates in the preset region; if the actual distance between the magnet and the reed switch is larger than the preset distance, the reed switch is actuated to open the circuit between the pH sensor 301 and the sample circuit 302 of the internal transmitting apparatus 30, and the external recording apparatus 20 detects that the internal transmitting apparatus 30 is not in the preset region.

In addition, the external recording apparatus 20 periodically detects the intensity of the signal received by the second wireless transceiver module 204, under the control of the second micro-processor 201, and if it is detected that the signal intensity is not in the preset range of signal intensity, the second micro-processor 201 controls the buzzer 209 and/or the status light 208 to alert. According to the invention, the external recording apparatus 20 further comprises a temperature sensor 202; and the storage 205 pre-stores the first pH calibration data of the internal transmitting apparatus 30; and the temperature sensor 202 detects the current room temperature, and sends the temperature data to the second micro-processor 201; and the second wireless transceiver module 204 receives the initialized data from the internal transmitting apparatus 30 and sends the data to the second micro-processor 201; and the initialized data can be the voltage difference.

The second micro-processor 201 calibrates the initialized data, and conducts temperature compensation in the course of calibration, in order to obtain the current calibration data; after that the second micro-processor 201 compares the current calibration data with the first pH calibration data pre-stored in the storage 205; if the current calibration data are the same as the first pH calibration data, the subsequent procedure is executed; if the current calibration data differ from the first pH calibration data, a calibration alerting signal is sent to the internal transmitting apparatus 30 through the second wireless transceiver module 204. And then the first wireless transceiver module 304 of the internal transmitting apparatus 30 receives the calibration alerting signal and send the signal to the first micro-processor 303; and the first micro-processor 303 controls the work light 306 to alert.

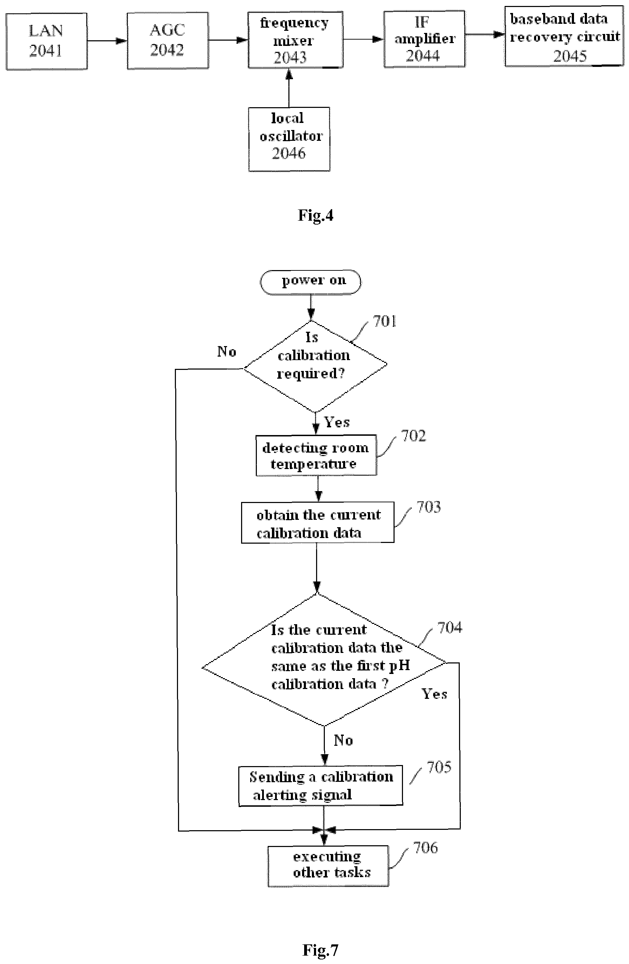

The second wireless transceiver module in Fig.4 comprises a low noise amplifier LNA 2041, an automatic gain control circuit AGC 2042, a frequency mixer 2043, a local oscillator 2046, an IF amplifier 2044, and a baseband data recovery circuit 2045. Wherein, the low noise amplifier 2041 may amplify the weak signal, so as to facilitate signal receiving thereafter; the AGC circuit 2042 can automatically adjust the gain of LNA 39 in terms of the detection of the signal intensity, and receive the signal with broader band; and the frequency mixer 2043 can be adopted to produce intermediate-frequency signal by mixing the frequency of the external high-frequency signal and that of the local signal, so as to facilitate data demodulation thereafter; the local oscillator 2046 may consist of a PLL circuit, which synthesize the local crystal oscillator signal into the signal with the frequency required by the frequency mixer 2043; the IF amplifier 2044 may be in the form of an intermediate-frequency filter amplifier, which processes the intermediate-frequency signal produced by the frequency mixer 2043, so as to facilitate the data demodulation thereafter; the baseband data recovery circuit 2045 may comprise a detecting circuit, a data filtering circuit, and a data shaping and recovery circuit, which demodulates low-frequency Analog signal.

With reference to Fig.2 and Fig.3, the invention provides a positioning system for wireless monitoring of esophageal pH value comprising an internal transmitting apparatus 30, and an external recording apparatus 20; wherein, the internal transmitting apparatus 30 comprises a pH sensor 301, a sample circuit 302, a first micro-processor 303, a power management unit 305, a first wireless transceiver module 304, and a first means 307; and the external recording apparatus 20 comprises a second micro-processor 201, a power management unit 210, a buzzer 209, a storage 205, a data interface 206, a key 207, a second wireless transceiver module 204, a status light 208, a housing 211, and a second means 203.

The second means 203 of the external recording apparatus 20 cooperates with the first means 307 of the internal transmitting apparatus 30; and if the external recording apparatus 20 detects that the internal transmitting apparatus 30 is not in the preset region, the second micro-processor 201 controls the buzzer 209 and/or status light 208 to alert.

The external recording apparatus 20 periodically detects the intensity of the signal received by second wireless transceiver module 204, under the control of the second micro-processor 201, and if it is detected that the signal intensity is not in the preset range of signal intensity, the micro-processor 201 controls the buzzer 209 and/or the status light 208 to alert.

The first means 307 of the internal transmitting apparatus 30 may be a permanent magnet; and the second means 203 of the external recording apparatus 20 may be a magnetic sensor; and the cooperation between the second means 203 of the external recording apparatus 20 and the first means 307 of the internal transmitting apparatus 30 comprises the fact that the intensity of magnetic field generated by the permanent magnet of the internal transmitting apparatus is detected through the magnetic sensor, and if the intensity of magnetic field is not in the preset range of magnetic intensity, the external recording apparatus 20 detects that the internal transmitting apparatus 30 is not in the preset region. The first means 307 of the internal transmitting apparatus 30 may be a reed switch in series with the pH sensor 301 and the sample circuit 302 and arranged therebetween; and the second means 203 of the external recording apparatus 20 may be a magnet, which actuates the reed switch through magnetic induction; wherein, the cooperation between the second means 203 of the external recording apparatus 20 and the first means 30 of the internal transmitting apparatus 30 specifically refers to the fact that if the actual distance between the magnet and the reed switch is no larger than the preset distance, the reed switch is actuated to close the circuit between the pH sensor 301 and the sample circuit 302 of the internal transmitting apparatus 30, and the external recording apparatus 20 detects that the internal transmitting apparatus 30 locates in the preset region; if the actual distance between the magnet and the reed switch is larger than the preset distance, the reed switch is actuated to open the circuit between the pH sensor 301 and the sample circuit 302 of the internal transmitting apparatus 30, and the external recording apparatus 20 detects that the internal transmitting apparatus 30 is not in the preset region.

Furthermore, according to the invention, the external recording apparatus 20 further comprises a temperature sensor 202; and the storage 205 pre-stores the first pH calibration data of the internal transmitting apparatus 30; and the temperature sensor 202 detects the current room temperature, and sends the temperature data to the second micro-processor 201; and the second wireless transceiver module 204 receives the initialized data from the internal transmitting apparatus 30 and sends the data to the second micro-processor 201; and the initialized data can be the voltage difference.

The second micro-processor 201 calibrates the initialized data, and conducts temperature compensation in the course of calibration, in order to obtain the current calibration data; after that the second micro-processor 201 compares the current calibration data with the first pH calibration data pre-stored in the storage 205; if the current calibration data are the same as the first pH calibration data, the subsequent procedure is executed; if the current calibration data differ from the first pH calibration data, a calibration alerting signal is sent to the internal transmitting apparatus 30 through the second wireless transceiver module 204.

In addition, the data recorder may alert through the buzzer and/or the status light 208 under the control of the second micro-process 201

The internal transmitting apparatus 30 further comprises work light 306; and the first wireless transceiver module 304 of the internal transmitting apparatus 30 receives the calibration alerting signal and sends the signal to the first micro-processor 303; and then the first micro-processor 303 controls the work light 306 to alert.

It is obvious that the positioning system described in the embodiments of this invention enables the real time monitoring of the position of the internal transmitting apparatus in the esophagus through the cooperation between the first means 307 of the internal transmitting apparatus 30 and the second means of the external recording apparatus 20, which not only avoids the invalid detection due to unexpected drop of the capsule, but also reduces the cost of detection for patients; in addition, the fact that the internal transmitting apparatus 30 is applied for detecting the intensity of the received signal may help to avoid signal interrupt problem caused when the patients are working or sleeping, which ensures the integrality of detection data; furthermore, the calibration of the system before being put in use and the temperature compensation in the course of calibration improve the testing accuracy.

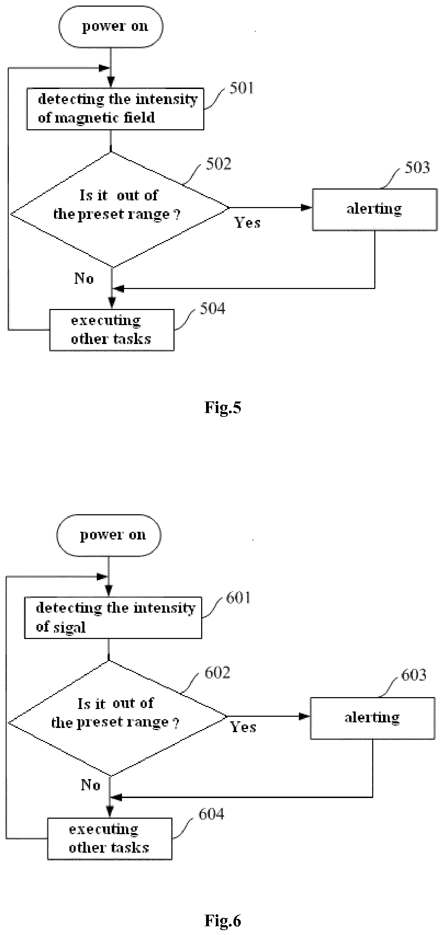

Fig. 5 illustrates a flow diagram of implementing the position of the internal transmitting apparatus the internal transmitting apparatus may be a pH capsule, the first means of the internal transmitting apparatus may be a permanent magnet, the external recording apparatus may be a data recorder, and the second means of the external recording apparatus may be a magnetic sensor.

The procedure of positioning may comprise:
step 501: allowing the data recorder to periodically detect the intensity of magnetic field generated by the permanent 307 in the capsule through the magnetic sensor 203 under the control of the second micro-processor 201;
step 502: determining if the intensity of the detected magnetic field of the capsule is in the preset range of magnetic intensity; and if it is in the preset range, executing step 503, otherwise, executing step 504;
step 503: allowing the data recorder to alert through the buzzer 209 and/or the status light 208, under the control of the second micro-processor 201, so as to indicate the failure of positioning;
step 504: ending the procedure, and executing the other tasks.

The other tasks of the system in this invention refer to the tasks other than the abovementioned task, and the phrase will also be used in the following embodiments of the specification with the same meaning.

Fig. 6 illustrates a flow diagram of implementing the alerting of communication failure; the internal transmitting apparatus may be a pH capsule, and the external recording apparatus may be a data recorder.

The procedure of implementing the alerting of communication failure comprises:
step 601: allowing the data recorder to periodically detect the intensity of signal received by the second transceiver module 204, under the control of the second micro-processor 201;
step 602: determining if the detected intensity of signal is in the preset range of signal intensity; if it is in the preset range, executing step 604, otherwise, executing step 603;
step 603: allowing the data recorder to alert through the buzzer 209 and/or the status light 208, under the control of the second micro-processor 201, so as to indicate the signal intensity failure;
step 604: ending the procedure, and executing the other tasks.

Fig. 7 illustrates a flow diagram of calibration of the system before being put in use; the internal transmitting apparatus may be a pH capsule, and the external recording apparatus may be a data recorder.

The procedure of calibration may comprise:
step 701: determining if there is a need for calibration in terms of the instruction received; if it is needed, executing step 702, otherwise, executing step 706;
step 702: detecting the room temperature through the temperature sensor 202 in the course of calibration;
step 703: receiving the initialized data from the internal transmitting apparatus 30, calibrating the initialized data, and conducting temperature compensation in the course of the calibration, in order to obtain the current calibration data; wherein, the temperature compensation specifically refers to detecting the room temperature, and conducting temperature compensation for the calibrated pH-mV curve by conducting table lookup and processing with software, so as to minimize the influence of temperature on the testing.
step 704: determining the validity of the current calibration data, more specifically, which refers to determining if the current calibration data is the same as the first pH calibration data pre-stored; if they are the same, executing step 706, otherwise, executing step 705;
step 705: sending the calibration alerting signal to the internal transmitting apparatus; and meanwhile, allowing the data recorder to alert through the buzzer 209 and/or status light 208, under the control of the second micro-processor 201; for example, the buzzer sounds every 1 second, in order to notify the operator;
step 706: ending the procedure, and executing the other tasks.

One embodiment of this invention provides a positioning method for wireless monitoring of esophageal pH value, based on Fig.5 and Fig.7, comprising:
determining if the internal transmitting apparatus 30 locates in the preset region, through the cooperation between the recording apparatus 20 and the internal transmitting apparatus 30, and alerting if the internal transmitting apparatus 30 is not in the preset region; and determining if the signal intensity is in the preset range of signal intensity, based on the intensity of the received signal, which is periodically detected by the external recording apparatus 20, and alerting if the signal intensity is not in the preset range of signal intensity.

Furthermore the position method, before the system is put in use, further comprising: pre-storing the first pH calibration data of the internal transmitting apparatus 30 in the external recording apparatus 20; and
calibrating the initialized data received by the external recording apparatus 20 from the internal transmitting apparatus 30, and conducting temperature compensation in the course of calibration in order to obtain the current calibrated data;
comparing the current calibrated data with the first pH calibration data pre-stored, and sending the calibration alerting signal to the internal transmitting apparatus if the current calibrated data differ from the first pH calibration data; and
controlling the internal transmitting apparatus to alert after the internal transmitting apparatus receives the calibration alerting signal. This embodiment of the invention provides a position method for wireless monitoring of esophageal pH value, wherein, the cooperation between the first means 307 of the internal transmitting apparatus 30 and the second means of the external recording apparatus 20 enables the real time monitoring of the position of the internal transmitting apparatus in the esophagus 1, which not only avoids the invalid detection due to unexpected drop of the capsule, but also reduces the cost of detection for patients; in addition, the fact that the internal transmitting apparatus 30 is applied for detecting the intensity of the signal received may help to avoid signal interrupt problem caused when the patients are working or sleeping, which ensures the integrality of detection data; furthermore, the calibration of the system before being put in use and the temperature compensation in the course of calibration improve the testing accuracy.

It will be noted that the term "comprises/comprising" as used in this description is intended to denote the presence of a given characteristic, step or component, without excluding the presence of one or more other characteristic, features, integers, steps, components or groups thereof. While this invention has been described as having a preferred design, the present invention can be further modified within the scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. Positioning system for wireless monitoring of esophageal pH value, comprising:
an internal transmitting apparatus (30), and
an external recording apparatus (20);
wherein,
said internal transmitting apparatus (30) comprises a pH sensor (301), a sample circuit (302), a first micro-processor (303), a power management unit (305), a first wireless transceiver module (304), and a first means (307);
said external recording apparatus (20) comprises a second micro-processor (201), a power management unit (210), a buzzer (209), a storage (205), a data interface (206), a key (207), a second wireless transceiver module (204), a status light (208), a housing (211), and a second means (203); and
said second means (203) of said external recording apparatus (20) is configured to cooperate with said first means (307) of said internal transmitting apparatus (30); and if said external recording apparatus (20) detects that said internal transmitting apparatus (30) is not in the preset region, said micro-processor (201) is configured to control said buzzer (209) and/or status light (208) to alert;
said external recording apparatus (20) is configured to periodically detect the intensity of the signal received by said second wireless transceiver module (204), under the control of said micro-processor (201), and if it is detected that the signal intensity is not in the preset range of signal intensity, said micro-processor (201) is configured to control said buzzer (209) and/or said status light (208) to alert; **characterized in that** said external recording apparatus (20) further comprises temperature sensor (202); and
said storage (205) is configured to pre-store the first pH calibration data of said internal transmitting apparatus (30); and
said temperature sensor (202) is configured to detect the current room temperature, and to send the temperature data to said second micro-processor (201); and
said second wireless transceiver module (204) is configured to receive the initialized data from said internal transmitting apparatus (30) and to send the data to said second micro-processor (201); and
said second micro-processor (201) is configured first to calibrate said initialized data, and to conduct temperature compensation in the course of calibration, in order to obtain the current calibration data; then to compare said current calibration data with said first pH calibration data pre-stored in said storage (205); and to send a calibration alerting signal to said internal transmitting apparatus (30) through second wireless transceiver module (204) if said current calibration data differ from said first pH calibration data; and
said internal transmitting apparatus (30) further comprises a work light (306); and
said first wireless transceiver module (304) of said internal transmitting apparatus (30) is configured to receive said calibration alerting signal and send the signal to said first micro-processor (303); and
said first micro-processor (303) is configured to control said work light (306) to alert.

2. The system of claim 1, wherein,
said first means (307) of said internal transmitting apparatus (30) is a permanent magnet; said second means (203) of said external recording apparatus (20) is a magnetic sensor; the cooperation between said second means (203) of said external recording apparatus (20) and said first means (307) of said internal transmitting apparatus (30) comprises the fact that the intensity of magnetic field generated by said permanent magnet of said internal transmitting apparatus (30) is detected through said magnetic sensor, and if said intensity of magnetic field is not in the preset range of magnetic intensity, said external recording apparatus (20) is configured to detect that said internal transmitting apparatus (30) is not in the preset region.

3. The system of claim 1, wherein,
said first means (307) of said internal transmitting apparatus (30) is a reed switch in series with said pH sensor (301) and said sample circuit (302) and arranged therebetween;
said second means (203) of said external recording apparatus (20) is a magnet, which is configured to actuate said reed switch through magnetic induction;
the cooperation between said second means (203) of said external recording apparatus (20) and said first means (30) of said internal transmitting apparatus (30) comprises the fact that if the actual distance between said magnet and said reed switch is larger than the preset distance, said reed switch is actuated to open the circuit between said pH sensor (301) and said sample circuit (302) of said internal transmitting apparatus (30), and said external recording apparatus (20) is configured to detect that said internal transmitting apparatus (30) is not in the preset region.

4. A positioning method for wireless monitoring of esophageal pH value, by means of an external recording apparatus (20) and an internal transmitting apparatus (30), wherein, comprising
determining if said internal transmitting apparatus (30) locates in the preset region, through the cooperation between said recording apparatus (20) and said internal transmitting apparatus (30), and alerting if said internal transmitting apparatus (30) is not in the preset region; and determining if the signal intensity is in the preset range of signal intensity, based on the intensity of the received signal, which is periodically detected by said external recording apparatus (20), and alerting if said signal intensity is not in the preset range of signal intensity **characterized in that**, before the system is put in use, the position method further comprises
pre-storing said first pH calibration data of said internal transmitting apparatus (30) in said external recording apparatus (20); detecting room temperature by a temperature sensor in the external recording apparatus (20); and calibrating the initialized data received by said external recording apparatus (20) from said internal transmitting apparatus (30), and conducting temperature compensation in the course of calibration, in order to obtain the current calibrated data; comparing said current calibrated data with said first pH calibration data pre-stored, and sending a calibration alerting signal to said internal transmitting apparatus (30) if said current calibrated data differ from said first pH calibration data; and
controlling said internal transmitting apparatus (30) to alert after said internal transmitting apparatus (30) receives said calibration alerting signal.

## Patentansprüche

1. Positionierungssystem zur drahtlosen Überwachung des Speiseröhren-pH-Wertes, umfassend:
einen internen Sendeapparat (30), und
einen externen Aufzeichnungsapparat (20);
wobei
der interne Sendeapparat (30) einen pH-Sensor (301), eine Abtastschaltung (302), einen ersten Mikroprozessor (303), eine Energieverwaltungseinheit (305), ein erstes drahtloses Sende-/Empfängermodul (304) und ein erstes Mittel (307) umfasst;
der externe Aufzeichnungsapparat (20) einen zweiten Mikroprozessor (201), eine Energieverwaltungseinheit (210), einen Summer (209), ein Speichermittel (205), eine Datenschnittstelle (206), eine Taste (207), ein zweites drahtloses Sende-/Empfängermodul (204), eine Statusleuchte (208), ein Gehäuse (211) und ein zweites Mittel (203) umfasst; und
das zweite Mittel (203) des externen Aufzeichnungsapparats (20) so konfiguriert ist, dass es mit dem ersten Mittel (307) des internen Sendeapparats (30) zusammenwirkt; und wenn der externe Aufzeichnungsapparat (20) detektiert, dass sich der interne Sendeapparat (30) nicht in der vorgewählten Region befindet, der Mikroprozessor (201) konfiguriert ist, um den Summer (209) und/oder die Statusleuchte (208) so zu steuern, dass Alarm gegeben wird;
der externe Aufzeichnungsapparat (20) konfiguriert ist, um unter Steuerung des Mikroprozessors (201) die Intensität des Signals periodisch zu detektieren, welches von dem zweiten drahtlosen Sende-/Empfängermodul (204) empfangen wird, und falls detektiert wird, dass die Signalintensität nicht in dem vorgewählten Bereich der Signalintensität ist, der Mikroprozessor (201) konfiguriert ist, um den Summer (209) und/oder die Statusleuchte (208) so zu steuern, dass Alarm gegeben wird;
**dadurch gekennzeichnet, dass**
der externe Aufzeichnungsapparat (20) ferner Temperatursensor (202) umfasst; und
das Speichermittel (205) so konfiguriert ist, dass die ersten pH-Kalibrierungsdaten des internen Sendeapparats (30) vorab gespeichert werden; und
der Temperatursensor (202) so konfiguriert ist, dass die aktuelle Raumtemperatur detektiert wird und die Temperaturdaten an den zweiten Mikroprozessor (201) gesendet werden; und
das zweite drahtlose Sende-/Empfängermodul (204) so konfiguriert ist, dass die initialisierten Daten von dem internen Sendeapparat (30) empfangen werden und die Daten an den zweiten Mikroprozessor (201) gesendet werden; und
der zweite Mikroprozessor (201) so konfiguriert ist, dass zuerst die initialisierten Daten kalibriert werden und im Verlauf der Kalibrierung Temperaturkompensation durchgeführt wird, um die aktuellen Kalibrierungsdaten zu erhalten; dann die aktuellen Kalibrierungsdaten mit den ersten pH-Kalibrierungsdaten verglichen werden, die in dem Speichermittel (205) vorab gespeichert sind; und
ein Kalibrierungsalarmsignal über das zweite drahtlose Sende-/Empfängermodul (204) an den internen Sendeapparat (30) gesendet wird, wenn sich die aktuellen Kalibrierungsdaten von den ersten pH-Kalibrierungsdaten unterscheiden; und
der interne Sendeapparat (30) ferner eine Arbeitsleuchte (306) umfasst; und
das erste drahtlose Sende-/Empfängermodul (304) des internen Sendeapparats (30) konfiguriert ist, um das Kalibrierungsalarmsignal zu empfangen und das Signal an den ersten Mikroprozessor (303) zu senden; und
der erste Mikroprozessor (303) konfiguriert ist, um die Arbeitsleuchte (306) so zu steuern, dass Alarm ausgegeben wird.

2. System nach Anspruch 1, wobei:
das erste Mittel (307) des internen Sendeapparats (30) ein Permanentmagnet ist;
das zweite Mittel (203) des externen Aufzeichnungsapparats (20) ein magnetischer Sensor ist;
das Zusammenwirken zwischen dem zweiten Mittel (203) des externen Aufzeichnungsapparats (20) und dem ersten Mittel (307) des internen Sendeapparats (30) die Tatsache umfasst, dass die Intensität des Magnetfelds, welches durch den Permanentmagneten des internen Sendeapparats (30) generiert wird, durch den magnetischen Sensor detektiert wird, und
falls die Intensität des Magnetfeldes nicht in dem vorgewählten Bereich der magnetischen Intensität liegt, der externe Aufzeichnungsapparat (20) konfiguriert ist, um zu detektieren, dass der interne Sendeapparat (30) nicht in der vorgewählten Region ist.

3. System nach Anspruch 1, wobei:
das erste Mittel (307) des internen Sendeapparats (30) ein Reedschalter in Reihe mit dem pH-Sensor (301) und der Abtastschaltung (302) und zwischen diesen angeordnet ist;
das zweite Mittel (203) des externen Aufzeichnungsapparats (20) ein Magnet ist, der konfiguriert ist, um den Reedschalter mittels magnetischer Induktion zu betätigen;
das Zusammenwirken zwischen dem zweiten Mittel (203) des externen Aufzeichnungsapparats (20) und dem ersten Mittel (30) des internen Sendeapparats (30) die Tatsache umfasst, dass, wenn der tatsächliche Abstand zwischen dem Magneten und dem Reedschalter größer als der vorgewählte Abstand ist, der Reedschalter betätigt worden ist, um die Schaltung zwischen dem pH-Sensor (301) und der Abtastschaltung (302) des internen Sendeapparats (30) zu öffnen, und der externe Aufzeichnungsapparat (20) konfiguriert ist, um zu detektieren, dass der interne Sendeapparat (30) nicht in der vorgewählten Region ist.

4. Positionierungsverfahren zum drahtlosen Überwachen des Speiseröhren-pH-Wertes mittels eines externen Aufzeichnungsapparats (20) und eines internen Sendeapparats (30), welches umfasst:
Bestimmen, ob der interne Sendeapparat (30) sich in der vorgewählten Region befindet, durch das Zusammenwirken zwischen dem Aufzeichnungsapparat (20) und dem internen Sendeapparat (30), und Auslösen von Alarm, wenn der interne Sendeapparat (30) nicht in der vorgewählten Region ist; und Bestimmen, ob die Signalintensität in dem vorgewählten Bereich der Signalintensität ist, basierend auf der Intensität des empfangenen Signals, welches periodisch durch den externen Aufzeichnungsapparat (20) detektiert wird, und Auslösen von Alarm, falls die Signalintensität nicht in dem vorgewählten Bereich der Signalintensität ist, **dadurch gekennzeichnet, dass** das Positionierungsverfahren ferner umfasst, bevor das System in Betrieb genommen wird:
Vorabspeichern der ersten pH-Kalibrierungsdaten des internen Sendeapparats (30) in dem externen Aufzeichnungsapparat (20);
Detektieren der Raumtemperatur durch einen Temperatursensor in dem externen Aufzeichnungsapparat (20), und
Kalibrieren der initialisierten Daten, die durch den externen Aufzeichnungsapparat (20) von dem internen Sendeapparat (30) empfangen wurden, und
Durchführen von Temperaturkompensation im Verlauf der Kalibrierung, um die aktuellen kalibrierten Daten zu erhalten;
Vergleichen der aktuellen kalibrierten Daten mit den ersten vorab gespeicherten pH-Kalibrierungsdaten, und Senden eines Kalibrierungsalarmsignals an den internen Sendeapparat (30), wenn sich die aktuellen kalibrierten Daten von den ersten pH-Kalibrierungsdaten unterscheiden; und
Steuern des internen Sendeapparats (30), so dass Alarm ausgelöst wird, nachdem der interne Sendeapparat (30) das Kalibrierungsalarmsignal empfangen hat.

## Revendications

1. Système de positionnement pour la surveillance sans fil de la valeur du pH de l'oesophage, comprenant :
un appareil de transmission interne (30), et
un appareil d'enregistrement externe (20) ;
ledit appareil de transmission interne (30) comprenant un capteur de pH (301), un circuit d'échantillonnage (302), un premier micro-processeur (303), une unité de gestion d'alimentation (305), un premier module émetteur-récepteur sans fil (304) et un premier moyen (307) ;
ledit appareil d'enregistrement externe (20) comprenant un second micro-processeur (201), une unité de gestion d'alimentation (210), un vibreur (209), une mémoire (205), une interface de données (206), une clé (207), un second module émetteur-récepteur sans fil (204), une lumière d'état (208), un logement (211) et un second moyen (203) ; et
ledit second moyen (203) dudit appareil d'enregistrement externe (20) étant configuré pour coopérer avec ledit premier moyen (307) dudit appareil de transmission interne (30) ; et si ledit appareil d'enregistrement externe (20) détecte que ledit appareil de transmission interne (30) n'est pas dans la région prédéfinie, ledit micro-processeur (201) est configuré pour commander ledit vibreur (209) et/ou la lumière d'état (208) pour émettre une alerte ;
ledit appareil d'enregistrement externe (20) étant configuré pour détecter périodiquement l'intensité du signal reçu par ledit second module émetteur-récepteur sans fil (204), sous la commande dudit micro-processeur (201), et s'il est détecté que l'intensité de signal n'est pas dans la plage prédéfinie d'intensité de signal, ledit micro-processeur (201) est configuré pour commander ledit vibreur (209) et/ou ladite lumière d'état (208) pour émettre une alerte ;
**caractérisé en ce que**
ledit appareil d'enregistrement externe (20) comprend en outre un capteur de température (202) ; et
ladite mémoire (205) est configurée pour pré-stocker les premières données d'étalonnage de pH dudit appareil de transmission interne (30) ; et
ledit capteur de température (202) est configuré pour détecter la température ambiante actuelle,
et pour envoyer les données de température audit second micro-processeur (201) ; et
ledit second module émetteur-récepteur sans fil (204) est configuré pour recevoir les données initialisées provenant dudit appareil de transmission interne (30) et pour envoyer les données audit second micro-processeur (201) ; et
ledit second micro-processeur (201) est configuré d'abord pour étalonner lesdites données initialisées,
et pour réaliser une compensation de température au cours de l'étalonnage, afin d'obtenir les données d'étalonnage actuelles ; puis pour comparer lesdites données d'étalonnage actuelles avec lesdites premières données d'étalonnage de pH pré-stockées dans ledit stockage (205) ; et envoyer un signal d'alerte d'étalonnage audit appareil de transmission interne (30) par l'intermédiaire d'un second module émetteur-récepteur sans fil (204) si lesdites données d'étalonnage actuelles diffèrent desdites premières données d'étalonnage de pH ; et
ledit appareil de transmission interne (30) comprend en outre une lumière de travail (306) ; et
ledit premier module émetteur-récepteur sans fil (304) dudit appareil de transmission interne (30) est configuré pour recevoir ledit signal d'alerte d'étalonnage et envoyer le signal audit premier micro-processeur (303) ; et
ledit premier micro-processeur (303) est configuré pour commander ladite lumière de travail (306) pour émettre une alerte.

2. Système selon la revendication 1,
ledit premier moyen (307) dudit appareil de transmission interne (30) étant un aimant permanent ;
ledit second moyen (203) dudit appareil d'enregistrement externe (20) étant un capteur magnétique ;
la coopération entre ledit second moyen (203) dudit appareil d'enregistrement externe (20) et ledit premier moyen (307) dudit appareil interne de transmission (30) comprenant le fait que l'intensité du champ magnétique généré par ledit aimant permanent dudit appareil de transmission interne (30) est détectée à travers ledit capteur magnétique, et si ladite intensité de champ magnétique n'est pas dans la plage prédéfinie d'intensité magnétique, ledit appareil d'enregistrement externe (20) est configuré pour détecter que ledit appareil de transmission interne (30) n'est pas dans la région prédéfinie.

3. Système selon la revendication 1,
ledit premier moyen (307) dudit appareil de transmission interne (30) étant un commutateur à lames en série avec ledit capteur de pH (301) et ledit circuit d'échantillonnage (302) étant disposé entre ceux-ci ;
ledit second moyen (203) dudit appareil d'enregistrement externe (20) étant un aimant qui est configuré pour actionner ledit commutateur à lames par induction magnétique ;
la coopération entre ledit second moyen (203) dudit appareil d'enregistrement externe (20) et ledit premier moyen (30) dudit appareil de transmission interne (30) comprenant le fait que si la distance réelle entre ledit aimant et ledit commutateur à lames est supérieure à la distance prédéfinie, ledit commutateur à lames est actionné pour ouvrir le circuit entre ledit capteur de pH (301) et ledit circuit d'échantillonnage (302) dudit appareil de transmission interne (30), et ledit appareil d'enregistrement externe (20) est configuré pour détecter que ledit appareil de transmission interne (30) n'est pas dans la région prédéfinie.

4. Procédé de positionnement pour la surveillance sans fil de la valeur du pH de l'oesophage, au moyen d'un appareil d'enregistrement externe (20) et d'un appareil de transmission interne (30), comprenant :
la détermination du fait que ledit appareil de transmission interne (30) se situe dans la région prédéfinie, par l'intermédiaire de la coopération entre ledit appareil d'enregistrement (20) et ledit appareil de transmission interne (30), et l'émission d'une alerte si ledit appareil de transmission interne (30) n'est pas dans la région prédéfinie ; et
la détermination du fait que l'intensité du signal est dans la plage prédéfinie d'intensité de signal, sur la base de l'intensité du signal reçu, qui est périodiquement détecté par ledit appareil d'enregistrement externe (20), et l'émission d'une alerte si ladite intensité de signal n'est pas dans la plage prédéfinie d'intensité de signal, **caractérisée en ce que**, avant la mise en oeuvre du système, le procédé de positionnement comprend en outre :
le pré-stockage desdites premières données d'étalonnage de pH dudit appareil d'émission interne (30) dans ledit appareil d'enregistrement externe (20) ; la détection de la température ambiante par un capteur de température dans l'appareil d'enregistrement externe (20) ; et
l'étalonnage des données initialisées reçues par ledit appareil d'enregistrement externe (20) à partir dudit appareil de transmission interne (30), et la réalisation d'une compensation de température au cours de l'étalonnage, afin d'obtenir les données étalonnées actuelles ;
la comparaison desdites données étalonnées actuelles avec lesdites premières données d'étalonnage de pH pré-stockées, et l'envoi d'un signal d'alerte d'étalonnage audit appareil de transmission interne (30) si lesdites données étalonnées actuelles diffèrent desdites premières données d'étalonnage de pH ; et
la commande dudit appareil de transmission interne (30) pour émettre une alerte après que ledit appareil de transmission interne (30) ait reçu ledit signal d'alerte d'étalonnage.
